# EUROPÄISCHE PATENTANMELDUNG

(11) **EP 1 217 098 A1**
(43) Veröffentlichungstag der Anmeldung: **26.06.2002**
(21) Anmeldenummer: 01128811.5
(22) Anmeldetag: 04.12.2001
(51) Int. Cl.: C25B 9/00, C25B 11/02, C07C 245/02

(54) **Bipolare quasigeteilte Elektrolysezellen**

(30) Priorität: 19.12.2000 DE 10063195
(71) Anmelder: BASF AKTIENGESELLSCHAFT, 67056 Ludwigshafen (DE)
(72) Erfinder: Merk, Claudia, Dr., 67117 Limburgerhof (DE); Brudermüller, Martin, Dr., 68239 Mannheim (DE); Pütter, Hermann, Dr., 67433 Neustadt (DE)

(57) **Zusammenfassung**

Elektrolysezelle bestehend aus 2 monopolaren Elektroden und einer oder mehreren dazwischenliegenden bipolaren Elektroden, wobei
- die eine monopolare Elektrode und die hierzu gleichsinnig geladenen Teile der bipolaren Elektroden gemeinsam die Arbeitselektrode bilden und die andere monopolare Elektrode und die hierzu gleichsinnig geladenen Teile der bipolaren Elektroden gemeinsam die Gegenelektrode bilden
- der Raum zwischen Gegen- und Arbeitselektrode ungeteilt ist
- die Oberfläche der Gegenelektrode aus elektrochemisch aktiven und inaktiven Teilen besteht
- die Summe der elektrochemisch aktiven Teile der Oberfläche der Gegenelektrode um ein vielfaches kleiner ist als die der elektrochemisch aktiven Teile der Oberfläche der Arbeitselektrode.

## Beschreibung

Die vorliegende Erfindung betrifft eine Elektrolysezelle bestehend aus 2 monopolaren Elektroden und einer oder mehreren dazwischenliegenden bipolaren Elektroden, wobei
- die eine monopolare Elektrode und die hierzu gleichsinnig geladenen Teile der bipolaren Elektroden gemeinsam die Arbeitselektrode bilden und die andere monopolare Elektrode und die hierzu gleichsinnig geladenen Teile der bipolaren Elektroden gemeinsam die Gegenelektrode bilden
- der Raum zwischen Gegen- und Arbeitselektrode ungeteilt ist
- die Oberfläche der Gegenelektrode aus elektrochemisch aktiven und inaktiven Teilen besteht
- die Summe der elektrochemisch aktiven Teile der Oberfläche der Gegenelektrode um ein vielfaches kleiner ist als die der elektrochemisch aktiven Teile der Oberfläche der Arbeitselektrode.

Weiterhin betrifft die Erfindung Verfahren zur Herstellung von organischen und anorganischen Verbindungen unter Anwendung der vorgenannten Elektrolysezellen, insbesondere die Herstellung von Azodicarbonsäure-di-(C₁-bis C₆-alkyl)estern.

Elektrolysezellen werden in der modernen Chemie in einer Vielfalt von Gestaltungen für eine Vielzahl von Aufgaben eingesetzt. Ein Überblick über die Konstruktionsmöglichkeiten von Elektrolysezellen findet sich z.B. in D. Pletcher, F. Walsh, Industrial Electrochemistry, 2. Auflage, 1990, London 60ff. Eine Übersicht über industrielle, elektrochemische Prozesse bietet der Artikel von D. Degner, Topics in Current Chemistry, 148,1ff, 1988. Eine häufig benutzte und auch technisch eingesetzte Form von Elektrolysezellen ist die Plattenstapelzelle oder Kapillarspaltzelle (vgl. Ullmann's Encyclopedia of Industrial Chemistry, sixth Edition, 2000 Electronic Release, Chapter 5.4.3.2. "Cell Design"). Häufig werden bei der Anordnung der Kapillarspaltzelle, die Elektroden und entsprechende Trennelemente filterpressenartig angeordnet und durch Abstandsmedien wie Abstandshalter oder Diaphragmen getrennt. Eine sogenannte ungeteilte Zelle beinhaltet meist nur eine Elektrolytphase, eine geteilte Zelle weist zwei oder mehrere solcher Phasen auf. In der Regel sind die den Elektroden benachbarten Phasen flüssig. Es können aber auch sogenannte Festkörperelektrolyten wie Ionenaustauschermembranen als Elektrolytphasen eingesetzt werden. Wird die Elektrode dabei direkt auf die Ionenaustauschermembran aufgebracht z.B. in Form einer elektrokatalytischen und feinporösen Schicht, so sind zusätzlich Kontaktierungen nötig, die einerseits als Stromkollektoren, andererseits als Stofftransportförderer ausgebildet sein müssen. Die einzelnen Elektroden können parallel (monopolar) oder seriell (bipolar) geschaltet werden.

Um einen möglichst hohen Stoffumsatz in Elektrolysezellen zu erreichen, soll der Elektrolyt nach allgemeiner Lehre derart an die Elektroden herangeführt werden, dass ein optimaler Stofftransport erreicht wird. Bei Flüssigelektrolyten wird häufig vorgeschlagen, die Elektrolytflüssigkeit parallel zu den Elektroden strömen zu lassen.

Die Raum-Zeit-Ausbeute und die Selektivität der Elektrolyse hängen neben der Anströmung der Elektroden auch von den verwendeten Elektrodenmaterialien ab. Diese beeinflussen Haltbarkeit, Größe, und Gewicht der Zelle maßgeblich.

In bekannten Plattenstapelzellen werden die Elektroden überwiegend als massive Platten, z.B. Graphitscheiben eingesetzt.

Diese werden auch vor allem, in den oben erwähnten technischen Prozessen eingesetzt, wie z.B. in der Synthese von Anisaldehyddimethylacetal in DE 2 848 397, Tolylaldehyddimethylacetal in EP 129795 oder zur Herstellung von α-Hydroxymethylketalen wie in EP 0460451 beschrieben ist.

Derartige Elektroden haben vielfältige Nachteile, die sich aus der Massivität des Materials ergeben, z.B. die gegenüber einem porösen Material verminderte Oberfläche und dem damit einhergehenden verminderten Stoffumsatz, der sich in geringeren Stromausbeuten bemerkbar macht, höherem Gewicht und einem größeren Raumbedarf. Weiterhin entspricht durch die bipolare Schaltung der massiven Elektroden, die Anode in der Größe der Fläche der Kathode.

In diesen Fällen bieten sogenannte dreidimensionale Elektroden eine Möglichkeit zur Erhöhung des Stofftransports und somit eine Möglichkeit die Stromausbeute zu steigern.

Ansätze zur Optimierung der Flächenverhältnisse sind z.B. in DE 19533773 beschrieben, hier wurde eine Filzanode großer Oberfläche mit einer flächigen Kathode kombiniert. Dabei unterscheiden sich die Elektroden in Ihrer Oberfläche dadurch, dass bei der Gegenelektrode nur die dem Elektrolyt zugewandte Fläche als aktive Oberfläche wirkt, während die Arbeitselektrode vom Elektrolyt durchströmt werden kann. Aus den von den Herstellern gelieferten Oberflächengewichten lassen sich für die eingesetzten Filze Oberflächenverhältnisse Arbeitselektrode zu Gegenelektrode von 1,2 bis 2,4 berechnen.

Ebenso beschreiben Montiel et al. (Ind. Eng. Chem. Res. 1998, 37 (11), 4501-11) den Einsatz von Graphitfilzen in einer Filterpresse, um die Oberfläche der Arbeitselektrode zu vergrößern.

Oftmals kann es jedoch vorkommen, dass Anodenraum und Kathodenraum getrennt werden müssen, dies ist vorzugsweise dann der Fall, wenn chemische Nebenreaktionen oder Rückreaktionen auszuschließen sind oder wenn nachfolgende Stofftrennungen zu vereinfachen sind. Weiterhin kann dies der Fall sein, wenn eine Substanz sowohl leicht oxidierbar als auch reduzierbar ist, so dass ein sogenannter Elektronenshuttle-Prozess vorliegt. Wird keine Trennung von Anoden- und Kathodenraum vorgenommen, steigt die verbrauchte Menge an Ladung an und es kommt ggf. zu unerwünschten Nebenreaktionen.

Ein Beispiel für eine Reaktion, die aufgrund von Nebenreaktionen/Rückreaktionen bisher in einer geteilten Zelle gefahren werden musste, ist die z.B. in FR 02043109 (DT 2016764) beschriebene Oxidation von Hydrazodicarbonsäureamiden- oder Hydrazodicarbonsäureestern zu den entsprechenden Azoverbindungen. Die Trennung der Elektrolysekreisläufe wird hier durch ein Diaphragma oder eine Membran gelöst.

In diesem Fall kommen in der Regel geteilte Zellen zum Einsatz. Hier wird häufig mit planparalleler Elektrodenanordnung oder kerzenförmigen Elektroden gearbeitet. Als Trennmedium können Ionenaustauschermembranen, mikroporöse Membranen, Diaphragmen, Filtergewebe aus nichtelektronenleitenden Materialien, Glasfritten sowie poröse Keramiken eingesetzt werden. Der Aufbau solcher Zellen ist jedoch relativ kompliziert. Weiterhin werden Ionenaustauschermembranen, insbesondere Kationenaustauschermembranen, verwendet, vorzugsweise solche, die aus einem Copolymer aus Tetrafluorethylen und einem perfluorierten Monomer, das Sulfogruppen enthält, bestehen. Diese leitfähigen Membranen sind handelsüblich unter dem Handelsnamen Nafion ® (Fa. E.T. DuPont de Nemours and Company) und Gore Select ® (Fa. W. L. Gore & Associates, Inc.) erhältlich.

Der Einsatz dieser Membranen stößt oftmals an seine Grenzen, sobald in organischen Lösungsmitteln gearbeitet wird. Die Membranen quellen auf und sind für einen weiteren Einsatz in der Elektrolyse nicht geeignet.

Als Alternative zu den soeben beschriebenen geteilten Zellen können hier sogenannte quasigeteilte oder pseudogeteilte Zellen eingesetzt werden, die bisher vor allem im Labormaßstab ihre Anwendung finden. Das Prinzip, das diesem Zelltyp zugrunde liegt, besteht darin, dass die Arbeitselektrode eine deutlich größere Oberfläche aufweist als die entsprechende Gegenelektrode.

Untersuchungen hierzu wurden z.B. von Hamzah und Kuhn (Chem.-Ing.-Tech. 52, (1980), 762-763) durchgeführt. Es wurde der Einfluß des Flächenverhältnisses von Anode und Kathode auf die Bildung von Hypochlorit aus Chlorid ermittelt, wobei die Flächenverhältnisse von 0,76/1 bis 1,8 zu 1 variiert wurden.

Von E. Steckhan et al. Tetrahedron, 52, 1996, 9743-9754 wurde eine Elektrolysezelle beschrieben, bei der eine Drahtelektrode mit einer flächigen oder dreidimensional durchströmbaren Elektrode, auch in Form einer zylindrischen Anordnung kombiniert wird.

Birkett et al. beschreiben in Electrochimica Acta 27, 1982, 263-268 die Anwendung einer monopolaren Plattenrahmenzelle, die erhebliche Flächenunterschiede zwischen Anode und Kathode aufweist. Den Flächenunterschied erreichen Birkett et al. dadurch, dass sie Elektroden mit Lack besprühen und die Oberfläche so durch eine nichtleitfähige Schicht isolieren. Alternativ dazu wurde durch Auflage eines dichten Polypropylengewebes die Oberfläche einer massiven zweidimensionalen Elektrode deutlich verkleinert. Der maximale Flächenunterschied lag bei 2,7 cm² für die Gegenelektrode und 200 cm² für die Arbeitselektrode. Nachteilig sind hier die monopolare Fahrweise und die vergleichsweise hohen Spannungen, die diese Systeme zeigen.

Eine großtechnisch relevante quasigeteilte Zelle ist in WO 95/07375 von EA-Technology beschrieben. Hier wird ein Graphitfilz großer Oberfläche als Kathode eingesetzt, welcher eine DSA-Streckmetallelektrode als Anode entgegengesetzt wird.

Nachteilig an diesen Konstruktionen ist jedoch, dass aufgrund der speziellen räumlichen Anordnung von Arbeits- und Gegenelektrode die Reaktionen eine ungünstige Raum-Zeit-Ausbeute aufweisen.

Die technische Aufgabe bestand somit darin, einen im Vergleich zu geteilten Zellen relativ einfach aufgebauten Zelltyp bereitzustellen, in dem die gezielte Herstellung chemischer Verbindungen mit hoher Ausbeute, bezogen auf die eingesetzte Ladungsmenge und Menge an Ausgangsverbindungen, hoher Selektivität sowie hoher Raum-Zeit-Ausbeute möglich ist.

Der Aufbau der erfindungsgemäßen Elektrolysezelle erfolgt im allgemeinen nach der Art von Plattenstapelzelle oder Kapillarspaltzelle. Hierbei sind insbesondere die bipolaren Elektroden in Form von Flächenelementen wie Platten, Blechen, Scheiben, Vliesen, Folien oder sonstigen flächigen Gebilden parallel zueinander angeordnet.

Die Oberfläche der bipolaren Elektrode mit der einen Polarität bildet die Arbeitselektrode, wobei die Oberfläche mit der anderen Polarität die Gegenelektrode bildet. Dabei ist der elektrochemisch aktive Teil der Oberfläche der Gegenelektrode um ein vielfaches kleiner ist als der elektrochemisch aktive Teil der Oberfläche der Arbeitselektrode. Bevorzugt beträgt das Verhältnis aus aktiven Teilen der Oberfläche der Arbeitselektrode zu aktiven Teilen der Oberfläche der Gegenelektrode 1,5 : 1 bis 25 : 1, bevorzugt 2:1 bis 10:1. Dabei werden erfindungsgemäß aktive Teile der Oberfläche der Gegenelektrode inaktiviert. Das bedeutet, dass an diesen Teilen der Oberfläche praktisch keine elektrochemische Reaktion mehr stattfinden kann. Das Verhältnis aktiver Oberflächenteile zu inaktiven Oberflächenteilen beträgt im allgemeinen 0,01 : 1 (Restfläche:1%) bis 0,5 : 1 (50%), bevorzugt 0,03: 1 (3%) bis 0,3 :1 (30%), besonder bevorzugt 0,05 (5%): 1 bis 0,2 (20%): 1.

Als Material für die Arbeitselektrode kommen beispielsweise massive Materialien wie massives Graphit, Graphitpappe und insbesondere für den Elektrolyten durchströmbare Materialien wie Graphitfilzplatte, Kohlefilzplatte, Gewebe mit Kohlenstoff-bedeckter Elektrolytkontaktfläche, Kohlenstoffgewebe, Kohlenstoffnetze, andere poröser Festkörper aus Kohlenstoff, andere poröse Materialien, die nicht aus Kohlenstoff bestehen, die mit Kohlenstoff gefüllt sind oder an der Elektrolytkontaktfläche mit Kohlenstoff bedeckt sind, sowie poröse Metalle, z.B. Metallschwämme in Betracht. Solche Materialien sind z.B. in der DE-A-19533773 beschrieben.

Die durchströmbaren Materialien werden bevorzugt eingesetzt, weisen diese doch, bezogen auf den von ihnen benötigten Platzbedarf, eine besonders hohe elektrochemisch aktive Oberfläche auf.

Als Materialien für die Gegenelektrode eignen sich beispielsweise massives Graphit, Graphitpappe, massives Metall oder Metalloxid, massives Graphit, auf der Elektrolytkontaktfläche überzogen mit einer dünnen Schicht aus Metall, z.B. Metallfolie, massives Graphit, auf der Elektrolytkontaktfläche überzogen mit einer Kationen- oder Anionenaustauschermembran, die ggf. mit einem Katalysator beschichtet sind.

Für den Fall, dass Metalle oder Metalloxide Ihre Anwendung finden, kommen insbesondere Platin, Eisen/Eisenoxid, Nickel/Nickeloxid, Kupfer, Silber, Cadmium oder Blei/Bleidioxid in Betracht.

Metalle, insbesondere Metallfolien sind besonders bevorzugt, wenn die Gegenelektrode als Kathode geschaltet wird, da in solchen Fällen die Wasserstoffüberspannung auf ein Minimum reduziert wird. Hierdurch kann eine unerwünschte Rückreaktion und folglich das Elektronenshuttling vermieden werden und stattdessen wird Wasserstoff gebildet.

Als Ionenaustauschermembranen (elektrisch leitfähige Folien) eignen sich besonders zu Folien verarbeitete Polymere wie Polyethylen, Polyacrylate, Polysulfon und perfluorierte, Polymere mit negativ geladenen Gruppen wie Carboxylat- und Sulfonatgruppen (Kationenaustauschermembran) oder positiv geladenen Gruppen wie protonierten oder quaternierten Aminogruppen (Anionenaustauschermembran). Geeignete Kationenaustauscherpolymere sind beispielsweise anionische perfluorierte Polymere, die Sulfonatgruppen tragen. Solche Folien sind handelsüblich beispielsweise unter den Handelsnamen Nafion® (Fa. DuPont de Nemours and Company) und Gore Select® (Fa. W.L. Gore& Associates) erhältlich.
Als Katalysatoren, mit denen die Ionenaustauschermembran beschichtet sein kann, eigenen sich Platin-, Palladium- oder auch Nickelpulver sowie Aktivkohle, sowie Gemische daraus.

Die bipolaren Elektroden bestehen üblicherweise entweder aus Flächenelementen aus einem einzigen der o.g. Materialien, sofern das entsprechende Material in den beiden vorstehenden Listen für Arbeits- und Gegenelektrodenmaterialien aufgeführt ist.

Weiterhin können die bipolaren Elektroden aus 2 Flächenelementen aus 2 oder mehreren verschiedenen Materialen, von denen je eines aus einer der vorstehenden Listen für Arbeits- und Gegenelektrodenmaterialien ausgesucht ist, zusammengesetzt sein. In diesem Fall stehen die Flächenelemente in direktem Kontakt miteinander, d.h. sind sandwichartig zusammengesetzt.

Bevorzugt ist insbesondere die Kombination, bei der ein massives Material die Gegenelektrode und ein durchströmbares Material die Arbeitselektrode bildet.

Teile der Gegenelektrode werden im allgemeinen inaktiviert, indem man denen entsprechende aktive Teile der Oberfläche mit einem Lack oder einer Folie aus nichtleitendem Material wie Teflon, Polyethylen oder Polypropylen abdeckt (nachstehend "Maske" genannt).

Bevorzugt kommen als Masken gelochte Kunststoff-Folien zum Einsatz. Die Löcher in dieser besagten elektrisch nichtleitfähigen Kunststoff-Folie sind so angebracht, dass die Oberfläche der Elektrode auf Werte von 1-50% reduziert wird, weiterhin bevorzugt auf 3-30% und besonders bevorzugt auf 5-20%. Die Größe der Löcher ist im allgemeinen so bemessen, dass der Mittelwert der maximalen Abstände von 2 Punkten in einem Loch 0,05 mm bis 50mm, , besonders bevorzugt 2 mm bis 20 mm und vor allem bevorzugt 3 mm bis 10 mm beträgt. Im allgemeinen sind die Löcher statistisch verteilt. Die Form der noch aktiven Oberfläche ist allerdings nicht auf Löcher beschränkt. Sie kann ebenfalls zum Beispiel durch Streifen, spiralförmig zugeschnittene Folien oder andere Arten der Abdekkungsform erreicht werden, wichtig ist lediglich eine flächige Abdeckung, die die Größe der Oberfläche der Gegenelektrode im Vergleich zur Arbeitselektrode entscheidend verringert.

Die Dicke der Folie beträgt im allgemeinen 0,1µ bis 5 mm.

Bekanntlich werden die bipolare Elektrolysezelle mit parallelen Flächenelementen als bipolaren Elektroden im allgemeinen so betrieben, dass der Elektrolyt kontinuierlich parallel zu den Elektroden strömt und eine Strömung entlang des Potentialgefälles nach Möglichkeit unterbunden wird. Besonders effektiv kann diese unerwünschte Strömung dadurch behindert werden, dass Bestandteil der bipolaren Elektroden eine für den Elektrolyten schwer- oder undurchdringliche Schicht ist. Bei dieser Schicht handelt es sich beispielsweise um Metallfolie, vorzugsweise um Graphitpappe. Diese Maßnahmen können unabhängig von der Beschaffenheit des Elektrolyten getroffen werden.

Aus Gründen der Optimierung der Raum-Zeit-Ausbeute wählt man den Raum zwischen Arbeits- und Gegenelektrode möglichst klein und stapelt die bipolaren Elektroden bevorzugt aufeinander. In solchen Stapelelektrolysezellen können Gegen- und Arbeitselektrode von 2 sich gegenüberliegenden bipolaren Stapelelektroden jedoch nur soweit angenähert werden, dass sichergestellt ist, dass a) sich die aktiven Teile der Gegenelektrode und die Arbeitselektrode nicht berühren, um einen Kurzschluss zu verhindern und b) der o.g. Stofftransport des Elektrolyten noch möglich ist.

Die maximale Annäherung und insbesondere Stapelung der bipolaren Elektroden bei Erfüllung der o.g. Erfordernisse lässt sich mit 2 verschiedenen Ausführungsvarianten erreichen:

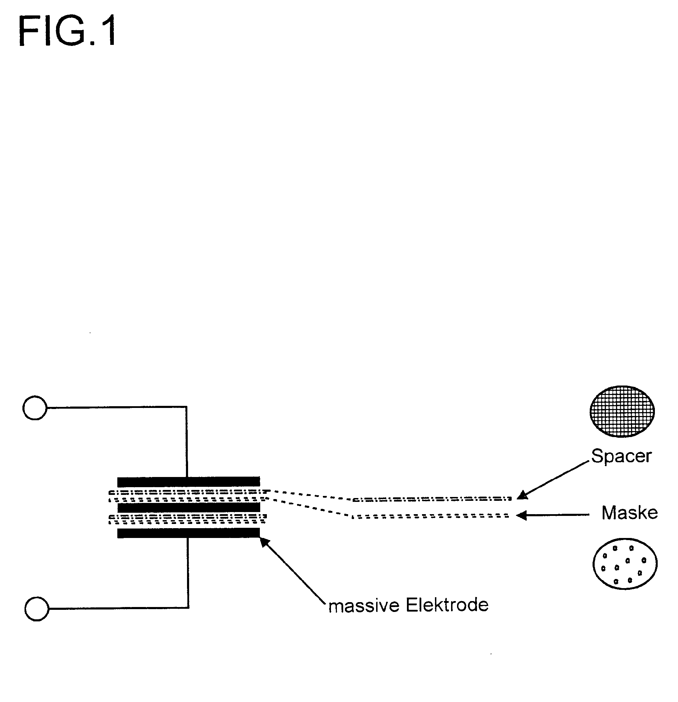

Zum einen ist es möglich, den Raum zwischen Arbeits- und Gegenelektrode teilweise mit einer Spacerschicht aus nichtleitendem Material auszufüllen, welches so angeordnet ist, dass dieser Raum vom Elektrolyten durchströmt werden kann, jedoch ein direktes In-Kontakt-Treten der Oberflächen von Arbeits- und Gegenelektrode verhindert wird (nachstehend "Spacer" genannt). Die Arbeitselektrode kann hierbei durchströmbar oder nicht durchströmbar sein. Die zweite Ausführungsform ist in Fig 1 abgebildet.

Der Spacer kann insbesondere bei Verwendung von massiven Elektroden unmittelbar auf diesen z.B. in Form von Noppen angebracht werden. Bevorzugt ist jedoch der Einsatz von Netzen oder Geweben Vliesen aus Kunststoffen, die als Gewebeformen gewebt, gestrickt, gestreckt und geprägt oder durch andere Formen zu Geweben verbunden werden, wobei die Art des Gewebes unkritisch ist, es muss sich lediglich um einen elektrischen Nichtleiter, wie z.B. Polyethylen, Polypropylen oder Teflon handeln.

Bei dieser Ausführungsvariante können alle vorgenannten Elektrodenmaterialien zu Einsatz kommen.

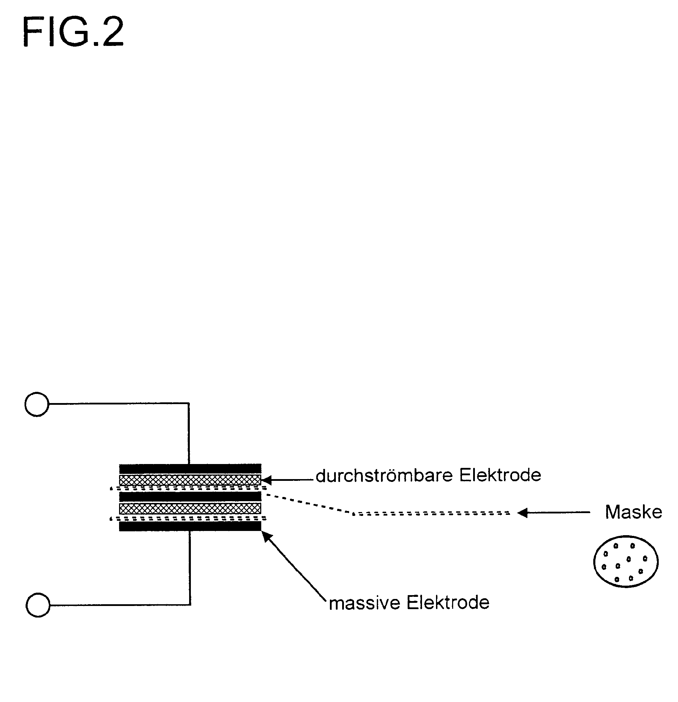

Nach einer zweiten Ausführungsvariante (vgl Fig 2) wird ohne Spacer gearbeitet.

In diesem Fall liegt die Oberfläche der Arbeitselektrode bevorzugt unmittelbar auf der Oberfläche der Maske der gegenüberliegenden Gegenelektrode auf. In diesem Fall besteht ein Raum zwischen Arbeits- und Gegenelektrode praktisch nur noch an den Stellen, an denen die Maske die Gegenelektrode nicht abdeckt. Um in diesem Fall sicherzustellen, dass in den Stapelelektroden noch ein Stofftransport parallel zu den bipolaren Elektroden möglich ist muss als Arbeitselektrode eine vom Elektrolyten durchströmbares Material gewählt werden.

Damit allerdings sichergestellt ist, dass sich das durchströmbare Material, welches die Arbeitselektrode bildet, nicht durch die Aussparungen der Maske auf die Gegenelektrode durchdrückt und so einen Kurzschluss verursacht empfiehlt es sich, als Maske eine Lochfolie zu verwenden, bei der der maximale Abstand von 2 Punkten in einem Loch .0,05mm. bis 5mm, bevorzugt 0,1mm bis 3mm, besonders bevorzugt 0,1 bis 0,5 mm beträgt. Bevorzugt weist die Folie eine Dicke von 0,1µm bis 3mm, bevorzugt 0,5 mm bis 1,5mm auf.

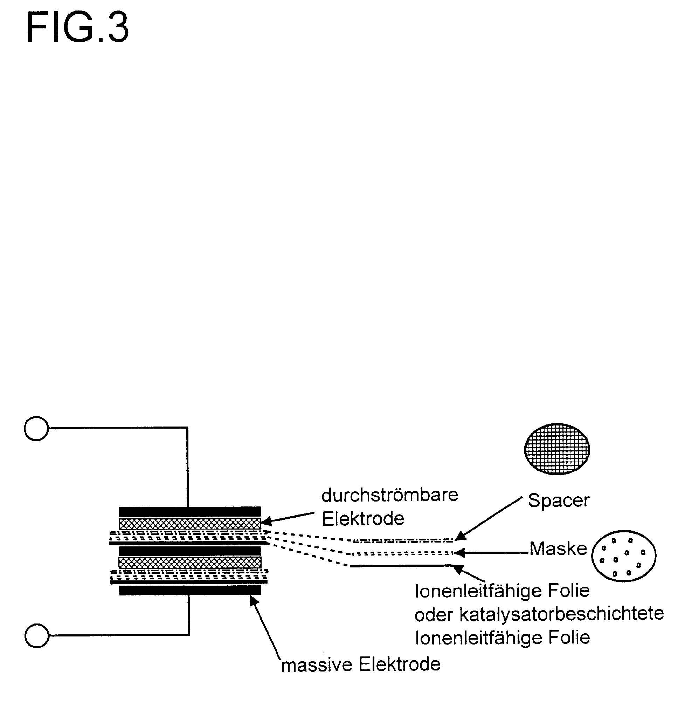

Im Falle der Verwendung von mit Ionenaustauschermembranen beschichteten Elektroden kommt meistens der in Fig 3 dargestellte Aufbau zur Anwendung.

Grundsätzlich gilt, dass die Arbeitselektrode jeweils Anode oder Kathode sein kann, woraus sich ergibt, dass sowohl die Kathode als auch die Anode als die in ihrer elektrochemisch aktiven Oberfläche eingeschränkte Gegenelektrode fungieren kann.

Durch die Reduzierung der Elektrodenoberfläche mit Hilfe eines elektrischen Nichtleiters kann die Spannung des Systems von ca. 3-6V/Spalt bis auf 20-30V/Spalt ansteigen. Dies hat zu Folge, dass ein Großteil der elektrischen Energie in Form von Wärme verlorengeht. Als Gegenmaßnahme kann die Leitfähigkeit des Elektrolyten erhöht werden, jedoch nur in dem Maße, wie die Bipolarität der Graphitscheiben noch erhalten werden kann. Als Anhaltspunkt kann man eine Verdopplung bis zu Verfünffachung der Konzentration des Elektrolyten annehmen.

Die Stromdichten liegen im erfindungsgemäßen Verfahren im allgemeinen ungefähr 100A/m² bis ungefähr 10 000 A/m², vorzugsweise ungefähr 300 A/m² bis 5000 A/m². Diese Werte beziehen sich auf die Fläche der Arbeitselektrode.

Das erfindungsgemäße Verfahren wird im allgemeinen bei Temperaturen von -10°C bis zum Siedepunkt des jeweils verwendeten Lösungsmittels verwendet, wobei Temperaturen von 5 bis 100°C, insbesondere 10 bis 60°C bevorzugt sind.

Das erfindungsgemäße Verfahren kann in Abhängigkeit der umzusetzenden Verbindung im saurem, d.h. bei einem pH-Wert, der unter 7, im neutralen, d.h. bei einem pH-Wert von ungefähr 7 und im basischen, d.h. bei einem pH-Wert, der über 7, Medium durchgeführt werden.

Insbesondere kann die erfindungsgemäße elektrochemische Reaktion entweder kontinuierlich oder diskontinuierlich durchgeführt werden.

Im allgemeinen wird die erfindungsgemäße elektrochemische Reaktion in Gegenwart eines Hilfselektrolyten vorgenommen. Neben der Einstellung der Leitfähigkeit der Elektrolyselösung dient der Hilfselektrolyt mitunter auch zur Steuerung der Selektivität der Reaktion.

Der Gehalt des Hilfselektrolyten liegt in der Regel bei einer Konzentration von ungefähr 0,1 bis ungefähr 10, vorzugsweise ungefähr 0,2 bis ungefähr 3 Gew.-% jeweils bezogen auf das Reaktionsgemisch. Als Hilfselektrolyt kommen Protonensäuren, wie z.B. organische Säuren, wobei Methansulfonsäure, Benzolsulfonsäure oder Toluolsulfonsäure genannt werden können und mineralische Säuren, wie z.B. Schwefelsäure, Chlor-, Brom- und Jodwasserstoffsäure und Phosphorsäure, in Betracht. Ferner können als Hilfselektrolyte auch Neutralsalze verwendet werden. Als Kationen kommen dabei Metallkationen von Lithium, Natrium, Kalium aber auch Tetraalkylammoniumkationen, wie z.B. Tetramethylammonuim, Tetraethylammonium, Tetrabutylammonium und Dibutyldimethylammonium in Frage. Als Anionen sind zu nennen: Fluorid, Tetrafluoroborat, Sulfonate, wie z.B. Methylsulfonat, Benzolsulfonat, Toluolsulfonat, Sulfate, wie z.B. Sulfat, Methylsulfat, Ethylsulfat, Phosphate, wie z.B. Methylphosphat, Dimethylphosphat, Diphenylphosphat, Hexafluorophosphat, Phosphonate, wie z.B. Methylphosphonatmethylester und Phenylphosphonatmethylester, aber auch die Halogenide Chlorid, Bromid und Iodid.

Weiterhin sind auch basische Verbindungen wie z.B. Alkali- oder Erdalkalimetallhydroxide, -carbonate, -hydrogencarbonate und - alkoholate einsetzbar, wobei Alkoholatanionen Methylat, Ethylat, Butylat und Isopropylat vorzugsweise eingesetzt werden.

Als Kationen kommen in diesen basischen Verbindungen wieder die oben genannten Kationen in Frage.

Weiterhin bevorzugt kann der eingesetzte Hilfselektrolyt auch elektrochemisch aktives Agens sein, so z.B. kann das aus Bromwasserstoffsäure gebildete Brom das elektrochemisch aktive Oxidans darstellen, welche die eigentliche Reaktion mit dem Substrat durchführt.

Als Lösungsmittel sind prinzipiell alle protischen Lösungsmittel, d.h. Lösungsmittel, die Protonen enthalten und freisetzen können und /oder Wasserstoffbrückenbindungen ausbilden können, wie z.B. Wasser, Alkohole, Amine, Carbonsäuren usw. ggf. im Gemisch mit aprotisch polaren Lösungsmitteln wie z.B. THF, 1,2-Dimethoxyethan, Dioxan im erfindungsgemäßen Verfahren geeignet.

Vorzugsweise werden dabei wegen der aufrecht zu erhaltenden Leitfähigkeit niedere Alkohole wie Methanol, Ethanol, iso-Propanol, n-Propanol, n-Butanol, sec-Butanol, tert-Butanol, Ether, wie z.B. Diethylether, 1,2-Dimethoxyethan, Furan, THF, Acetonitril und Dimethylformamid eingesetzt, vorzugsweise ein Gemisch dieser Lösungsmittel oder weiter bevorzugt Wasser im Gemisch mit diesen Lösungsmitteln in allen möglichen Mischungsverhältnissen.

Alternativ zu den oben erwähnten Alkoholen können auch deren Carbonsäuren oder Amide zum Einsatz kommen. Als Carbonsäuren werden bevorzugt eingesetzt, Ameisensäure, Essigsäure, Propionsäure und längerkettige verzweigte wie unverzweigte Carbonsäuren, weiterhin auch Schwefelsäure, Chlor-, Brom- und Jodwasserstoffsäure.

Die erfindungsgemäße Elektrolysezelle eignet sich grundsätzlich zur Herstellung beliebiger organischen und anorganische Verbindungen, die elektrochemisch herstellbar sind. Bevorzugt wird sie zur Herstellung solcher Verbindungen eingesetzt, bei denen sie in üblichen ungeteilten Zellen an der Gegenelektrode entweder zum Edukt zurückverwandelt werden oder in andere unerwünschte Produkte umgewandelt werden. Die Elektrolysezelle eignet sich weiterhin dazu, Redoxmediatoren in die gewünschte Oxidationsstufe zu überführen, in der diese mit einem Substrat unter dem Wechsel ihrer Oxidationsstufe abreagieren können.

Da in den folgenden Umsetzungen die Vorgehensweise nach den Verfahren des Standes der Technik mit mindestens einem der dargelegten Nachteile verbunden ist, lassen sich diese mit Vorteil in der erfindungsgemäßen Elektrolysezelle ausführen:

Zu den Reaktionen, die in ungeteilter Fahrweise in das Edukt zurückgeführt werden, gehören die Oxidation von Hydrazinen zu Diazoverbindungen, die Oxidation von Hydrochinonen zu Chinonen und umgekehrt, die Oxidation von organischen Iodverbindungen zu den Iodosoverbindungen bzw. mitunter auch zu den Bisacyloxy-iodverbindungen.

Zu den Reaktionen, die in ungeteilter Fahrweise zur Vernichtung des gebildeten Produkts führen, gehört die Reaktion von Aldehyden zu Carbonsäuren, welche in einer Konkurrenzreaktion Hydrodimerisieren oder zum Alkohol reduziert werden können. Ketone, die zu α-Hydroxyketalen umgesetzt werden, können in einer Konkurrenzreaktion ebenfalls hydrodimerisieren oder zum Alkohol reduziert werden. Bei der α-Oxidation von Ketonen zu α-Hydroxy- oder α-Formylestern, wie z.B. der Oxidation von Acetophenon zu Phenylglyoxylsäure, kann es ebenso zu Hydrodimerisierung oder Reduktion als Nebenreaktion kommen.

Weiterhin beobachtet man bei der Umsetzung von Hydroxylaminen, zu Aminen, eine Konkurrenzreaktion zu Oximen und weiteren Zersetzungsprodukten. Aromatische Nitroverbindungen, die zu Anilinderivaten umgesetzt werden, zeigen als Konkurrenzreaktion Zersetzungs- und Polymerisationsreaktionen. Wenn α-Nitrobenzoesäuren zu den entsprechenden Benzoxazolinonen umgesetzt werden, treten radikalische Zersetzungsreaktionen auf. Bei der Umsetzung von Hydrazinen ist eine Steuerung der Reaktion insofern erforderlich, als dass sowohl die Azoverbindung (s.o.), als auch Hydrazone oder eine Hydrazinspaltung die gewünschte Reaktion sein kann. In der Reduktion von Oxalsäure zu Glyoxylsäure kann mit diesem Verfahren die Weiteroxidation der Oxalsäure zu CO₂ verhindert werden. Bei der Enthalogenierung von Verbindungen soll auf diese Wiese die Polyhalogenierung durch freiwerdendes Halogen vermieden werden.

Im Falle von elektroenzymatischen Reaktionen ist das Ziel eine Zersetzung des Enzyms an der entsprechenden Elektrode gegebenenfalls zu vermeiden.

Zum dritten Fall, der Regenerierung von Mediatoren in dieser quasigeteilten Zelle, zählen anorganische Systeme, zum Beispiel, Ce(III/IV), Mn (II/III), Cr (II/III); Cr (III/VI); Ti (II/III); V(II/III); V(III/IV); V(IV/V); Ag(I/II); AgO⁺/AgO⁻; Cu (I/II); Sn(II/IV); Co (II/III); Mn (II/III); Mn(II/IV); Mn(II/VI); Os (IV/VIII); Os (III/IV); Os (IV/VI); Br₂/Br⁻/BrO₃; I₂, I⁻, IO₃⁻, oder auch organische Mediatoren wie ABTS, NAD⁺/NADH, NADP⁺/NADPH, TEMPO, Viologenen, wobei es sich bei den angegebenen Systemen auch um Metallkomplexe mit diversen Liganden oder auch Lösungsmittel-Liganden handeln kann.

Besonders eignet sich das die erfindungsgemäße Elektrolysezelle zur Herstellung von Azoverbindungen, insbesondere von Azodicarbonsäure-di-(C₁-bis C₆-alkyl)estern aus Hydrazodicarbonsäuredi-(C₁-bis C₆-alkyl)estern, wobei Azodicarbonsäurediethyl-, diisopropyl- oder di-tert.-butylester ganz besonders bevorzugt sind.

Wegen der geringen Stabilität der vorgenannten Azoverbindungen ist hier ein integriertes Gesamtverfahren mit einem integrierten Aufarbeitungsverfahren notwendig.

Über die thermische Zersetzung von Diazoamiden berichten J. E. Herweh et al., in J. Org. Chem.39, 1974, 786-793 und A.S. Prakash in J. Chem. Soc. Perkin II, 1975, 1, 46-50; vergleichbare Nebenprodukte werden bei den Diazoestern bereits bei Raumtemperatur in Gegenwart von Wasser bei den Elektrolyseausträgen beobachtet. In diesem Zusammenhang kann eine Zersetzungsreaktion von Azodicarbonsäureestern in wasserhaltigen Medien, in der Analogie von Wasser zu Alkoholen erklärt werden (F. Yoneda et al. beschreiben die Oxidation von Alkoholen zu den entsprechenden Ketonen mittels Azodicarbonsäureestern in Am. Soc. 1966, 88, 2328-2329).

Die mangelnde Stabilität der Azodicarbonsäuren ist auch bei deren Aufreinigung zu beachten, dies gilt insbesondere, wenn neben der Direktsynthese der Diazoester aus dem entsprechenden Hydrazin, ein elektrochemisches Recycling von genutztem Azoester durchgeführt wird.

Es wird üblicherweise folgendermaßen vorgegangen, um Zersetzungsreaktionen zu vermeiden:

Der Hydrazindicarbonsäureester wird in dem Elektrolyten gelöst und in einer oben beschriebenen Variante der quasigeteilten Kapillarspaltzelle umgesetzt. Bevorzugt sind inerte organische aprotische Lösungsmittel wie Ether, die ggf. in Abmischung mit protischen Lösungsmitteln eingesetzt werden. Werden in der Elektrolyse Wasser, Lösungsmittelgemische mit Wasser oder in C1-Stellung oxidierbare Alkohole als Reaktionsmedium eingesetzt, so sollte der gebildete Azoester rasch aus dem Reaktionsmedium entfernt werden, da sowohl Wasser, als auch Alkohole zur Zersetzung des Azoesters führen (s.o.). Der Elektrolyseaustrag wird sogleich in ein inertes, aprotisches organisches Medium wie z.B. Dieethylether, Methyl-tert.-butylether, Essigsäureethylester, Alkane, wie z.B. Pentan, n, i-Hexan, n, i-Heptan, Cyclohexan, Methylcyclohexan, Toluol, Dichlormethan u.a. gegeben welches nicht mit Wasser mischbar ist. Der entstandene Azoester wird so in die organische Phase extrahiert, in welcher er stabil vorliegt und kann nach Abtrennen der wäßrigen Phase nach üblichen Methoden weiterverarbeitet werden.

Beim Einsatz der Diazoester in der Mitsunobureaktion (O. Mitsunobu in Bull. Chem. Soc. Jpn. 1967, 40, 4235-4238 und Synthesis 1981, 1-28) liegen die umzusetzenden Hydrazodicarbonsäureester in Form einer Mischung mit Triphenylphosphinoxid vor. Um neben der Regenerierung des Hydrazodicarbonsäureesters eine Trennung dessen von Triphenylphosphinoxid zu erreichen geht man mit Vorteil so vor, dass man
- eine organisch-wässrige Lösung, enthaltend Hydrazodicarbonsäureester und Triphenylphosphinoxid zunächst einer Elektrolyse unterwirft und dabei den Hydrazodicarbonsäureester zu Azodicarbonsäureester umsetzt
- die Reaktionsmischung mit einem unpolaren Lösungsmittel extrahiert, in dem sich das Triphenylphosphinoxid nur schwer löst, z.B. Alkane, wie Pentan, n, i-Hexan, n, i-Heptan, Cyclohexan, Methylcyclohexan, oder Aromaten wie Toluol.
- die wässrige Phase mit einem in Wasser im wesentlichen unlöslichen organischen Lösungsmittel, in dem sich Triphenylphosphinoxid gut löst, z.B. Diethylether, Methyl-tert.-butylether, Essigsäureethylester oder Dichlormethan, extrahiert
- aus den beiden Extrakten nach üblichen Methoden den Azodicarbonsäureester und das Triphenylphosphinoxid isoliert.

### Experimenteller Teil

Oxidation von Hydrazodiisopropylcarbonsäureester zu Azodicarbonsäurediisopropylester

Vergleichsbeispiel 1: beide massiv

Der Plattenstapel bestand aus zwei massiven Ringscheiben aus Graphit, die im Abstand von 1mm voneinander angeordnet sind. Der Elektrolyt bestand aus 510 g Ethylenglykoldimethylether, 70g Wasser, 14,8g konzentrierter Schwefelsäure und 35g Hydrazodicarbonsäurediisopropylester und 81,7g Triphenylphosphinoxid.

Die Elektrolyse wurde bei einer Temperatur von 6°C und mit einer Stromdichte von 34 mA/cm² durchgeführt. Nach 4F liegt der Umsatz bei 42%, nach weiteren 2F ist ein Umsatz von 50% erreicht, das System ist in einem Gleichgewicht zwischen Produktbildung und Rückreaktion zum Edukt.

### Vergleichsbeispiel 2:

Fahrweise in einer geteilten Plattenrahmenzelle vom Durchflusstyp

| | |
|---|---|
| Membran | Nafion-324 |
| Anode | massiver Graphit 35cm² |
| Kathode | Edelstahl (V2A): 35cm² |

Der Katholyt bestand aus 73,5g Ethylenglykoldimethylether, 73,5g Wasser und 3,1g konzentrierter Schwefelsäure.

Der Anolyt bestand aus einem Gemisch von 88,1g Ethylenglykoldimethylether, 33,8g Wasser, 3,1g konzentrierter Schwefelsäure und 7,5g Hydrazodicarbonsäurediisopropylester und 17,5g Triphenylphosphinoxid.

Die Umsetzung wurde wie folgt durchgeführt: Zunächst wurden die beiden Zellkompartimente befüllt und auf 10°C abgekühlt. Die Stromdichte der Elektrolyse liegt bei 17mA/cm². Nach einer applizierten Ladungsmenge von 4F liegt der Umsatz bei 84%. Nach Aufarbeitung des Elektrolyseaustrages wird eine Ausbeute von 75% an Azodicarbonsäurediisopropylester (5,6g) erhalten.

### Vergleichsbeispiel 3:

Fahrweise in einer geteilten Plattenrahmenzelle vom Durchflusstyp

| | |
|---|---|
| Membran | Nafion-324 |
| Anode | Graphit 35cm² |
| Kathode | Edelstahl (V2A): 35cm² |

Der Katholyt bestand aus 73,5g Ethylenglykoldimethylether, 73,5g Wasser und 3,1g konzentrierter Schwefelsäure.

Der Anolyt bestand aus einem Gemisch von 86,6g Ethylenglykoldimethylether, 33,8g Wasser, 3,1g konzentrierter Schwefelsäure, 1,5g Natriumbromid, 7,5g Hydrazodicarbonsäurediisopropylester und 17,5g Triphenylphosphinoxid.

Die Umsetzung wurde wie folgt durchgeführt: Zunächst wurden die beiden Zellkompartimente befüllt und auf 10°C abgekühlt. Die Stromdichte der Elektrolyse liegt bei 17mA/cm². Nach einer applizierten Ladungsmenge von 4F liegt der Umsatz bei 95%. Nach Aufarbeitung des Elektrolyseaustrages wird eine Ausbeute von 92% an Azodicarbonsäurediisopropylester (6,9g) erhalten.

### Beispiel 1: ungeteilt in DME/Wasser - Filzanoden/Graphitkathoden und mit PTFE-abgedeckte Kathode (2,5% Restelektrodenfläche) dikker Filz

Der Plattenstapel bestand aus zwei massiven Ringscheiben aus Graphit und einem Graphitfilz des Typs RVG 2003 mit einer Dicke von 6mm. Als Abstandshalter diente ein Filternetz aus Polypropylen. Die Kathode, die aus einer massiven Graphitelektrode bestand, wurde mit einer Teflonfolie abgedeckt, die durch Löcher eines Durchmessers von 1,5mm die Elektrodenfläche auf 2,5% der ursprünglich eingesetzten Fläche reduziert. Der Elektrolyt bestand aus 383,9 g Ethylenglykoldimethylether, 210g Wasser, 14,6g konzentrierter Schwefelsäure, 7g Natriumbromid und 35g Hydrazodicarbonsäurediisopropylester und 47,4g Triphenylphosphinoxid.

Die Elektrolyse wurde bei einer Temperatur von 20°C und mit einer Stromdichte von 34 mA/cm² durchgeführt. Nach 4F ist Vollumsatz erreicht und nach Aufarbeitung wird eine Ausbeute von 84% an Azodicarbonsäurediisopropylester (29,06 g) erhalten.

### Beispiel 2: dünnerer Filz 2,5 %

Ein Wechsel der Anodenoberfläche zu einer dünneren Filzelektrode hat einen verringerten Umsatz und eine verringerte Selektivität zur Folge.

Der Plattenstapel bestand aus zwei massiven Ringscheiben aus Graphit und einem Graphitfilz des Typs KFD 2 mit einer Dicke von 2mm. Als Abstandshalter diente ein Filternetz aus Polypropylen.

Die Kathode, die aus einer massiven Graphitelektrode bestand, wurde mit einer Teflonfolie abgedeckt, die durch Löcher eines Durchmessers von 1,5mm die Elektrodenfläche auf 2,5% der ursprünglich eingesetzten Fläche reduziert. Der Elektrolyt bestand aus 436,4 g Ethylenglykoldimethylether, 157,5g Wasser, 14,6g konzentrierter Schwefelsäure, 7g Natriumbromid und 35g Hydrazodicarbonsäurediisopropylester und 47,4g Triphenylphosphinoxid.

Die Elektrolyse wurde bei einer Temperatur von 20°C und mit einer Stromdichte von 34 mA/cm² durchgeführt. Nach 4F liegt der Umsatz bei 79%. Nach Aufarbeitung wird eine Ausbeute von 63% an Azodicarbonsäurediisopropylester (21,81 g) erhalten.

### Beispiel 3: ungeteilt in DME/Wasser - Filzanoden/Graphitkathoden und mit PTFE-abgedeckte Kathode (10% Restelektrodenfläche).

Der Plattenstapel bestand aus zwei massiven Ringscheiben aus Graphit und einem Graphitfilz des Typs Carbone RVG 2003 mit einer Dicke von 6mm. Als Abstandshalter diente ein Filternetz aus Polypropylen. Die Kathode, die aus einer massiven Graphitelektrode bestand, wurde mit einer Teflonfolie abgedeckt, die durch Löcher eines Durchmessers von 3,0mm die Elektrodenfläche auf 10% der ursprünglich eingesetzten Fläche reduziert. Der Elektrolyt bestand aus 428,5 g Ethylenglykoldimethylether, 157,5g Wasser, 14,6g konzentrierter Schwefelsäure, 14,9g 47%iger Bromwasserstoffsäure und 35g Hydrazodicarbonsäurediisopropylester und 47,4g Triphenylphosphinoxid.

Die Elektrolyse wurde bei einer Temperatur von 20°C und mit einer Stromdichte von 34 mA/cm² durchgeführt. Nach 4F liegt der Umsatz bei 99%. Nach Aufarbeitung wird eine Ausbeute von 90% an Azodicarbonsäurediisopropylester (31,3 g) erhalten.

### Beispiel 4:

Der Plattenstapel bestand aus vier massiven Ringscheiben aus Graphit und drei Graphitfilzen des Typs Carbone RVG 2003 mit einer Dicke von 6mm. Als Abstandshalter diente ein Filternetz aus Polypropylen. Die Kathoden, die aus einer massiven Graphitelektrode bestanden, wurden mit einer Teflonfolie abgedeckt, die durch Löcher eines Durchmessers von 3,0mm die Elektrodenfläche auf 10% der ursprünglich eingesetzten Fläche reduziert. Der Elektrolyt bestand aus 428,5 g Ethylenglykoldimethylether, 157,5g Wasser, 14,6g konzentrierter Schwefelsäure, 14,9g 47%iger Bromwasserstoffsäure und 35g Hydrazodicarbonsäurediisopropylester und 47,4g Triphenylphosphinoxid.

Die Elektrolyse wurde bei einer Temperatur von 20°C und mit einer Stromdichte von 34 mA/cm² durchgeführt. Nach 5F liegt der Umsatz bei 100%. Nach Aufarbeitung wird eine Ausbeute von 90% an Azodicarbonsäurediisopropylester (31,4 g) erhalten.

### Beispiel 5: ungeteilt in DME/Wasser - Filzanoden/Graphitkathoden und mit PTFE-abgedeckte Kathode (20% Restelektrodenfläche)

Der Plattenstapel bestand aus zwei massiven Ringscheiben aus Graphit und einem Graphitfilz des Typs Carbone RVG 2003 mit einer Dicke von 6mm. Als Abstandshalter diente ein Filternetz aus Polypropylen. Die Kathoden, die aus einer massiven Graphitelektrode bestanden, wurden mit einer Teflonfolie abgedeckt, die durch Löcher eines Durchmessers von 3,0mm die Elektrodenfläche auf 20% der ursprünglich eingesetzten Fläche reduziert. Der Elektrolyt bestand aus 428,5 g Ethylenglykoldimethylether, 157,5g Wasser, 14,6g konzentrierter Schwefelsäure, 14,9g 47%iger Bromwasserstoffsäure und 35g Hydrazodicarbonsäurediisopropylester und 47,4g Triphenylphosphinoxid.

Die Elektrolyse wurde bei einer Temperatur von 20°C und mit einer Stromdichte von 34 mA/cm² durchgeführt. Nach 4F liegt der Umsatz lediglich bei 78%. Nach Aufarbeitung wird eine Ausbeute von 76% an Azodicarbonsäurediisopropylester (22,6 g) erhalten. In einem Wiederholungsversuch wurde eine Ladungsmenge von 6F eingesetzt. Damit kann Vollumsatz erreicht werden.Die isolierte Ausbeute beträgt dann mit 28,75 g 81 % an Azodicarbonsäurediisopropylester).

### Beispiel 6: ungeteilt in DME/Wasser - Filzanoden/Graphitkathoden und mit PTFE-abgedeckte Kathode (15% Restelektrodenfläche)

Der Plattenstapel bestand aus zwei massiven Ringscheiben aus Graphit und einem Graphitfilz des Typs Carbone RVG 2003 mit einer Dicke von 6mm. Als Abstandshalter diente ein Filternetz aus Polypropylen. Die Kathoden, die aus einer massiven Graphitelektrode bestanden, wurden mit einer Teflonfolie abgedeckt, die durch Löcher eines Durchmessers von 3,0mm die Elektrodenfläche auf 15% der ursprünglich eingesetzten Fläche reduziert. Der Elektrolyt bestand aus 428,5 g Ethylenglykoldimethylether, 157,5g Wasser, 14,6g konzentrierter Schwefelsäure, 14,9g 47%iger Bromwasserstoffsäure und 35g Hydrazodicarbonsäurediisopropylester und 47,4g Triphenylphosphinoxid.

Die Elektrolyse wurde bei einer Temperatur von 20°C und mit einer Stromdichte von 34 mA/cm² durchgeführt. Nach 4F liegt der Umsatz bei 80%, nach 4,5F liegt der Umsatz bei 90%. Nach Aufarbeitung wird eine Ausbeute von 76% an Azodicarbonsäurediisopropylester (26,5g) erhalten.
in den oben genannten Versuchen wurden vergleichbare Ausbeuten erhalten, wenn Dimethoxyethan durch tert.-Butanol oder Tetrahydrofuran ersetzt wurde.

Aufarbeitung eines Elektrolyseaustrages der Oxidation von Hydrazindicarbonsäurediisopropylester, HDDE zu Azodicarbonsäurediisopropylester, DIAD:

82g des Elektrolyseaustrags aus Beispiel 1 wurde mit 150 ml Wasser verdünnt und die Lösung anschließend mit 90 ml MTBE extrahiert, nach weiterem dreimaligem Waschen mit je 40 ml MTBE wurden die organischen Phasen vereinigt und vom Lösungsmittel befreit. Der am Rotationsverdampfer erhaltene Rückstand wurde in n-Heptan ausgerührt und das ausgefallene Triphenylphosphinoxid abgetrennt. Das so erhaltene Triphenylphosphinoxid hatte eine Reinheit von 97 %, der erhaltene Azodicarbonsäureester hatte eine Reinheit von 75 %.

### Abkürzungen:

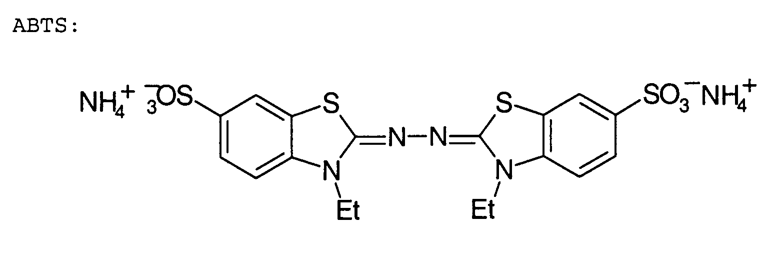

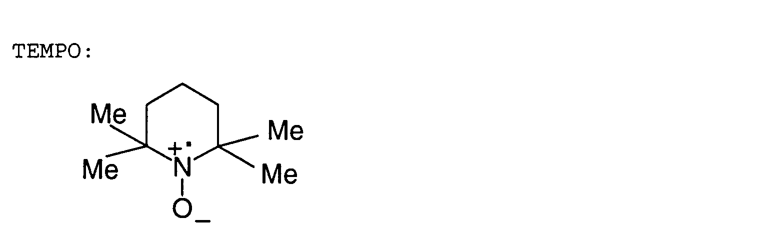

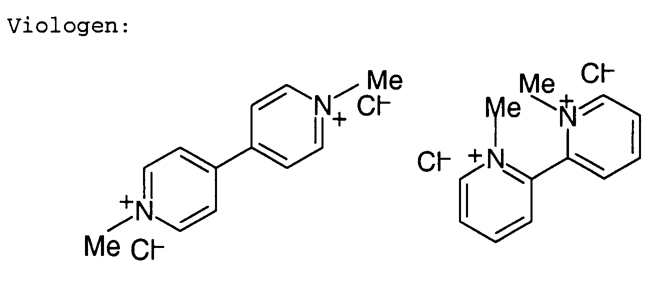

2,2'- und 4,4'-Methylviologene
MTBE: Methyl-tert.-butylether

## Patentansprüche

1. Elektrolysezelle bestehend aus 2 monopolaren Elektroden und einer oder mehreren dazwischenliegenden bipolaren Elektroden, wobei
- die eine monopolare Elektrode und die hierzu gleichsinnig geladenen Teile der bipolaren Elektroden gemeinsam die Arbeitselektrode bilden und die andere monopolare Elektrode und die hierzu gleichsinnig geladenen Teile der bipolaren Elektroden gemeinsam die Gegenelektrode bilden
- der Raum zwischen Gegen- und Arbeitselektrode ungeteilt ist
- die Oberfläche der Gegenelektrode aus elektrochemisch aktiven und inaktiven Teilen besteht
- die Summe der elektrochemisch aktiven Teile der Oberfläche der Gegenelektrode um ein vielfaches kleiner ist als die der elektrochemisch aktiven Teile der Oberfläche der Arbeitselektrode.

2. Elektrolysezelle nach Anspruch 1, **dadurch gekennzeichnet, dass** die Elektroden in Form von Platten, Blechen, Scheiben, Vliesen, Folien oder sonstigen flächigen Gebilden vorliegen und parallel zueinander angeordnet sind.

3. Elektrolysezelle nach Anspruch 1 oder 2 mit Gegenelektroden, bei denen man die elektrochemisch inaktiven Teile der Oberfläche, dadurch erzeugt, dass man die entsprechenden aktiven Teile der Oberfläche mit einem elektrisch nichtleitenden Material abdeckt.

4. Elektrolysezelle nach Anspruch 3, wobei die Abdeckung dadurch bewirkt wird, dass man auf die Gegenelektrode auf der Elektrolytkontaktfläche eine gelochte nichtleitende Kunststofffolie aufbringt.

5. Elektrolysezelle nach Anspruch 4, wobei die Größe der Löcher so bemessen ist, dass der Mittelwert der maximalen Abstände von 2 Punkten in einem Loch 0,05 bis 50 mm beträgt.

6. Elektrolysezelle nach den Ansprüchen 1 bis 5, wobei die Löcher statistisch oder nach einem bestimmten Muster verteilt sind.

7. Elektrolysezelle nach den Ansprüchen 1 bis 6, wobei 1 bis 75% der Gesamtfläche der Gegenelektroden elektrochemisch aktiv ist.

8. Elektrolysezelle nach den Ansprüchen 1 bis 7, wobei die Elektrolysezelle als Plattenstapelzelle oder Kapillarspaltzelle ausgeführt ist.

9. Elektrolysezelle nach den Ansprüchen 1 bis 8, wobei das Material, aus dem die Gegenelektrode hergestellt ist, aus folgender Gruppe ausgewählt ist: massives Graphit, Graphitpappe, massives Metall, massives Graphit, auf der Elektrolytkontaktfläche überzogen mit einer dünnen Schicht aus Metallfolie, massives Graphit, auf der Elektrolytkontaktfläche überzogen mit einer Kationen- oder Anionenaustauschermembran, die ggf. mit einem Katalysator beschichtet ist.

10. Elektrolysezelle nach den Ansprüchen 1 bis 9, wobei das Material, aus dem die Arbeitselektrode hergestellt ist, aus folgender Gruppe ausgewählt ist: massives Graphit, Graphitfilzplatte, Kohlefilzplatte, Gewebe mit Kohlenstoff-bedeckter Elektrolytkontaktfläche, poröser Festkörper, gefüllt mit Kohlenstoff, poröse Metalle, z.B. Metallschwämme.

11. Elektrolysezelle nach den Ansprüchen 1 bis 10, wobei der Raum zwischen Arbeits- und Gegenelektrode teilweise mit einer Spacerschicht aus nichtleitendem Material ausgefüllt ist, welches so angeordnet ist, dass dieser Raum vom Elektrolyten durchströmt werden kann, jedoch ein direktes In-Kontakt-Treten der Oberflächen von Arbeits- und Gegenelektrode verhindert wird.

12. Elektrolysezelle nach den Ansprüchen 1 bis 11, wobei die Spacerschicht in Kombination mit einer Arbeitselektrode aus massivem Material, das nicht vom Elektrolyten durchströmt werden kann, eingesetzt wird.

13. Elektrolysezelle nach den Ansprüchen 1 bis 11, wobei die Gegenelektrode mit einer gelochten Kunststofffolie abgedeckt ist, wobei die Größe der Löcher so bemessen ist, daß der maximale Abstand von 2 Punkten in einem Loch 0,05 mm bis 50 mm beträgt.

14. Elektrolysezelle nach den Ansprüchen 1 bis 13, wobei die Gegenelektrode mit einer gelochten Kunststofffolie abgedeckt ist, die 0,1 µm bis 5 mm dick ist.

15. Elektrolysezelle nach den Ansprüchen 1 bis 14 , wobei der Abstand von Gegen- und Arbeitselektrode 0,2-5 mm, bevorzugt 0,5-1,5 mm beträgt.

16. Elektrolysezelle nach den Ansprüchen 4 bis 10, wobei
- die Arbeitselektrode vom Elektrolyten durchströmbar ist
- die Gegenelektrode mit einer gelochten Kunststofffolie abgedeckt ist, die Löcher aufweist, deren Grösse so bemessen ist, daß der maximale Abstand von 2 Punkten in einem Loch 0,05 mm bis 5 mm, beträgt, und deren Dicke von 0,1 µm bis 3 mm beträgt
- die Arbeitselektrode mit der Kunststofffolie in Kontakt steht.

17. Verfahren zur Herstellung von organischen und anorganischen Verbindungen, indem man die entsprechenden Ausgangsprodukte in einer Elektrolysezelle gemäß den Ansprüchen 1 bis 16 umsetzt.

18. Verfahren zur Herstellung von Azodicarbonsäure-di-(C₁-bis C₆-alkyl)estern aus Hydrazodicarbonsäure-di-(C₁-bis C₆-alkyl)estern in einer Elektrolysezelle gemäß den Ansprüchen 1 bis 16.

19. Verfahren nach Anspruch 16 zur Herstellung von Azodiethylcarbonsäureester, Azodiisopropylcarbonsäureester oder Azoditert.-butylcarbonsäureester in einer Elektrolysezelle gemäß den Ansprüchen 1 bis 16.
